# EUROPEAN PATENT APPLICATION

(11) **EP 4 603 496 A1**
(43) Date of publication of application: **20.08.2025**
(21) Application number: 22962347.5
(22) Date of filing: 19.10.2022
(51) Int. Cl.: C07H 1/00, C07H 1/06, C07H 5/02

(54) **METHOD FOR PREPARING SUCRALOSE CRUDE PRODUCT BY USING ALCOHOL-WATER ALKALINE HYDROLYSIS SYSTEM**

(71) Applicant: Anhui Jinhe Industrial Co., Ltd., Chuzhou, Anhui 239200 (CN)
(72) Inventor: CHEN, Yongle, Chuzhou, Anhui 239200 (CN); QIU, Xin, Chuzhou, Anhui 239200 (CN); ZHAO, Chengcheng, Chuzhou, Anhui 239200 (CN); LI, Pengfei, Chuzhou, Anhui 239200 (CN)
(74) Representative: Ipside
(86) International application number: PCT/CN2022/126051
(87) International publication number: WO 2024/082156

(57) **Abstract**

The present invention provides a preparation method of a sucralose crude product by using an alcohol-water alkaline hydrolysis system, and relates to the technical field of fine chemical engineering. In the present invention, an aqueous solution of sucralose-6-acetate crude product is taken as a raw material, and is subjected to alkaline hydrolysis in an alkali metal hydroxide and methanol-water system, such that sucralose-6-acetate and chlorosucrose ester impurities (sucralose diester and tetrachlorosucrose-6-acetate) in the aqueous solution of sucralose-6-acetate crude product are both converted into sucralose, thereby significantly improving the yield of sucralose. In the present invention, separation is realized by carrying out multiple instances of concentration and multiple instances of extraction-back extraction in an ethyl acetate/water double-solvent system, such that fat-soluble impurities and water-soluble impurities are balanced in the system, and the phenomenon of saccharide coking during the concentration process can be avoided, thereby fully crystallizing sucralose in ethyl acetate, improving the yield of sucralose, efficiently separating inorganic salts from sucralose, and reducing the treatment cost of subsequent high-salinity wastewater.

## Description

### Technical Field

The invention relates to the technical field of fine chemical engineering, in particular to a preparation method of a sucralose crude product by using an alcohol-water alkaline hydrolysis system.

### Background of the Invention

Sucralose, also known as Splenda, is a new kind of sweetener using sucrose as a raw material, almost 600 times as sweet as sucrose. It has the characteristics of pure taste, no participation in metabolism, high sweetness, no energy, good stability, high safety , etc., is considered to be a "zero calories" sugar with the most application value in the 2first century, and is widely applied to a plurality of fields of foods, beverages, daily chemicals, medicines, etc.

At present, many reports are made on methods for preparing sucralose by using sucralose-6-acetate as a raw material, and mainly include organic amine catalysis methods (for example, Chinese patents CN101260127A, CN112771060A and CN101260127A), ion exchange resin catalysis methods (for example, Chinese patents CN112409419A and CN102336787A) and alkali catalysis methods, wherein the alkali catalysis methods are simple in operation, low in production cost and most widely applied.

Chinese patent CN113004345A discloses a method for continuously synthesizing sucralose, which takes dehydrated sucralose-6-acetate and anhydrous lower alcohol as raw materials in the presence of alkali metal hydroxide or alkali metal alkoxide, and adopts a reaction rectifying tower to prepare sucralose, wherein the conversion rate of the sucralose-6-acetate is 99.5-99.9%.

Chinese patent CN104004032A discloses a preparation method of sucralose by continuous deacetylation of sucralose-6-acetate, comprising the steps of carrying out deacetylation reaction on sucralose-6-acetate in an alcohol solvent and an alkaline catalyst (KOH, NaOH, sodium methoxide, sodium ethoxide, potassium methoxide, potassium ethoxide), and then rectifying to obtain a sucralose crude product, wherein the yield of the sucralose is 85.1-86.8%.

Chinese patent CN112805291A discloses a preparation method of sucralose, comprising the steps of dissolving sucralose-6-ethyl ester in methanol, adding calcium oxide to enable the sucralose-6-ethyl ester to generate deacylation reaction, and filtering to obtain a crude sucralose product solution, wherein the yield of the sucralose is 85-93%.

Chinese patent CN1814609A discloses a method for increasing the synthesis yield of sucralose, using azo reagent to catalyze, using acetic acid as acylating agent, and synthesizing sucrose into sucrose-6-ethyl ester in a proper solvent; then reacting with proper chlorinating reagent in aprotic polar solvent under the catalysis of trichloroacetonitrile to synthesize sucralose-6-ethyl ester; and finally, finishing alcoholysis of the sucralose-6-ethyl ester in a KOH/methanol system to synthesize the sucralose, wherein the yield is 63.2-68%.

Chinese patent CN101012250A discloses a preparation method of sucralose, the invention uses polymer-loaded organic tin as a catalyst, sucrose reacts with an acylation reagent to generate sucrose-6-ester with high chemical purity, then the sucrose-6-ester is chloridized and subjected to alkaline hydrolysis to obtain sucralose, wherein the alkaline hydrolysis is carried out in the presence of alkaline substances (sodium hydroxide and potassium hydroxide) and an organic solvent (methanol or ethanol), and the yield of the alkaline hydrolysis step is 59.7-75.2%.

Chinese patent CN103145772A discloses a preparation method of sucralose, which comprises the step of carrying out strong base deprotection on sucralose-6-ester to obtain sucralose, wherein a strong base deprotection reagent is sodium methoxide, sodium ethoxide, magnesium methoxide or magnesium ethoxide, and the yield is 77.1-86.8%.

However, the above methods for preparing sucralose by using high-purity sucralose-6-ester as a raw material generally has the problem of low yield of sucralose.

### Summary of the Invention

In view of this, the present invention provides a preparation method of a sucralose crude product by using an alcohol-water alkaline hydrolysis system, and the method provided by the present invention has a high sucralose yield.

In order to achieve the above object, the present invention provides the following technical solutions:

The invention provides a preparation method of a sucralose crude product by using an alcohol-water alkaline hydrolysis system, characterized by comprising the following steps:
(1) Extracting crude sucralose-6-acetate aqueous solution with ethyl acetate to obtain a first ethyl ester phase and a first aqueous phase respectively; concentrating the first ethyl ester phase to obtain syrup; dissolving the syrup in a methanol-water mixed solvent to obtain a sucralose-6-acetate methanol aqueous solution; the sucralose-6-acetate crude product water solution comprises sucralose-6-acetate, sucralose diester and tetrachlorosucrose-6-acetate;
(2) Mixing the sucralose-6-acetate methanol aqueous solution with an alkali metal hydroxide for alkaline hydrolysis reaction, and adjusting the pH value of the obtained reaction solution to be neutral to obtain a sucralose alkaline hydrolysis solution; concentrating the sucralose alkaline hydrolysis solution to obtain a sucralose crude product concentrate, mixing and dissolving the sucralose crude product concentrate and ethyl acetate, and performing solid-liquid separation to obtain a second ethyl ester phase;
(3) Washing the second ethyl ester phase with water to obtain a second aqueous phase and a third ethyl ester phase respectively; performing ethyl acetate extraction on the second aqueous phase to obtain a third aqueous phase and a fourth ethyl ester phase respectively; the fourth ethyl ester phase is recycled to step (2) for dissolving the crude sucralose concentrate;
(4) Mixing the third ethyl ester phase with water, and concentrating to obtain a third ethyl ester phase concentrate; dissolving the third ester phase concentrate in ethyl acetate to obtain a crude sucralose ethyl acetate solution; crystallizing the ethyl acetate solution of the sucralose crude product to obtain a sucralose crude product and a fifth ethyl ester phase respectively;
(5) Washing the fifth ethyl ester phase with water to obtain a fourth aqueous phase and a sixth ethyl ester phase respectively; the fourth aqueous phase is recycled to be mixed with the third ethyl ester in step (4); concentrating the sixth ethyl ester phase to obtain recovered ethyl acetate and sugar residues respectively.

Preferably, in step (1), the residual amount of ethyl acetate in the syrup is < 0.5 g/L.

Preferably, in step (1), the sucralose-6-acetate content in the first aqueous phase is < 0.1 g/L.

Preferably, in step (1), the concentration of methanol in the methanol-water mixed solvent is 10-60 wt%.

Preferably, in step (2), the pH value of the alkaline hydrolysis reaction is 12.0-12.5, the temperature is 0-20 °C, and the time is 0.5-2 h.

Preferably, in step (2), the water content in the crude sucralose concentrate is < 0.1 wt%.

Preferably, in step (2), the content of inorganic salts in the second ethyl ester phase is < 100 ppm.

Preferably, in step (3), the number of times of water washing is 5-7; the volume ratio of the second ethyl ester phase to the water-washed single-use water is 0.1-0.3: 1; combining the aqueous phases obtained by the first to second water washing to be used as a second aqueous phase;

The aqueous phase obtained by the second-seventh washing is used for washing the second ethyl ester phase in the next batch of sucralose crude product preparation process.

Preferably, in step (3), the sucralose content in the third aqueous phase is < 0.5 g/L.

Preferably, in step (4), the water content in the third ester phase concentrate is < 0.5 wt%.

Preferably, in step (4), the sugar degree of the ethyl acetate solution of the sucralose crude product is 40-60%.

Preferably, in step (4), the crystallization temperature is 40-60 °C and the time is 4-12 h.

Preferably, in step (5), the sucralose content in the sixth ethyl ester phase is < 0.1 g/L.

The invention provides a preparation method of a sucralose crude product by using an alcohol-water alkaline hydrolysis system. According to the method, alkali metal hydroxide is used as an alkaline hydrolysis reagent, alkaline hydrolysis is carried out in a methanol-water system, sucralose-6-acetate, sucralose diester and tetrachlorosucrose-6-acetate can be subjected to alkaline hydrolysis to generate corresponding sucralose and tetrachlorosucrose, tetrachlorosucrose is continuously dechlorinated to form sucralose, and the sucralose-6-acetate and impurities (sucralose diester and tetrachlorosucrose-6-acetate) in a first aqueous solution are converted into sucralose, so that the raw material conversion rate and the yield of sucralose are remarkably improved, compared with the method of preparing sucralose by alkaline hydrolysis using high-purity sucralose-6-acetate as the raw material, the conversion rate of the raw material and the yield of sucralose in the method provided by the present invention are significantly increased. The invention carries out alkaline hydrolysis in a methanol-water system, can improve the alkaline hydrolysis efficiency, can reduce the phenomena of uneven stirring and reduced mass transfer effect caused by precipitation of fat-soluble impurities in the cooling alkaline hydrolysis process, and provides the product yield. The method provided by the invention uses multiple concentration and multiple extraction-back extraction between ethyl acetate/water double-solvent systems for separation, so that fat-soluble impurities and water-soluble impurities are balanced in the systems, the phenomenon of sugar coking in the concentration process can be avoided, sucralose is fully crystallized in ethyl acetate, and the yield of sucralose is improved. Inorganic salts (such as sodium chloride, potassium chloride and ammonium chloride) are easily soluble in water and insoluble in ethyl acetate, more inorganic salts exist in the sucralose alkaline hydrolysis solution obtained after alkaline hydrolysis and pH value adjustment to be neutral, if ethyl acetate is used, sucralose can be extracted from the system at a heating or normal temperature, but the using amount of ethyl acetate is large, and the high-salt wastewater remaining after extraction needs to be subjected to evaporation and crystallization treatment to collect the inorganic salts. The method provided by the invention utilizes the characteristic that inorganic salts (such as sodium chloride, potassium chloride and ammonium chloride) are insoluble in ethyl acetate, directly concentrates the sucralose alkaline hydrolysis liquid to remove water and methanol solvent, then adds ethyl acetate to dissolve sucralose, water-soluble impurities and fat-soluble impurities, while the inorganic salts are insoluble in ethyl acetate, so that the inorganic salts are effectively separated from sucralose, and the subsequent treatment cost of high-salt wastewater is reduced.

### Brief Description of the Drawings

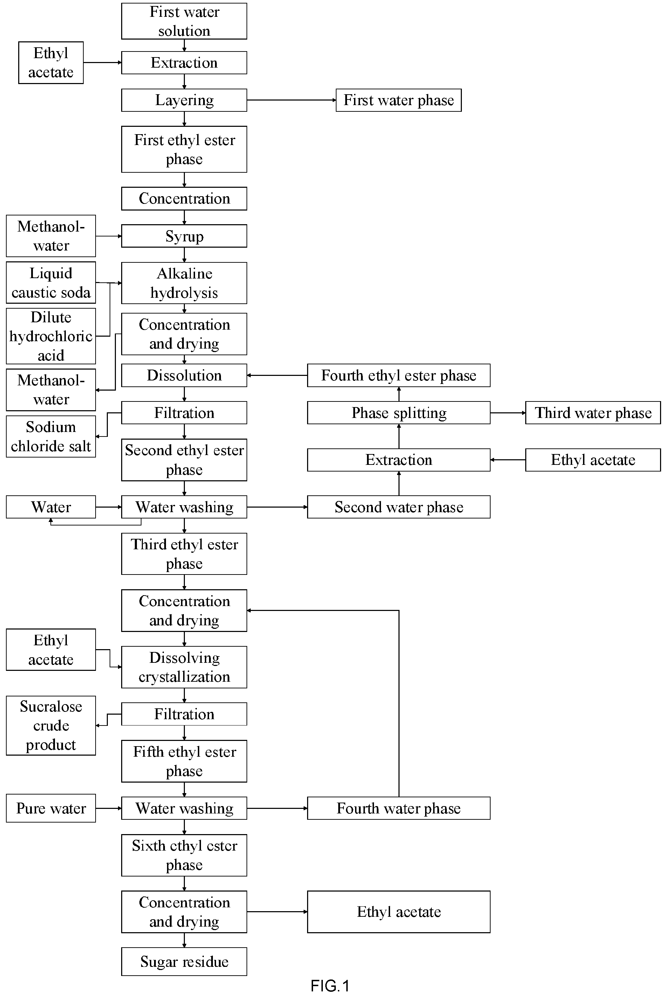

FIG. 1 is a flow chart of a process for preparing crude sucralose by using an alcohol-water alkaline hydrolysis system in example 1.

### Detailed Description of Embodiments

The invention is further illustrated below with reference to examples and figures.

The invention provides a preparation method of a sucralose crude product by using an alcohol-water alkaline hydrolysis system, which comprises the following steps:
(1) Extracting the crude sucralose-6-acetate aqueous solution with ethyl acetate to obtain a first ethyl ester phase and a first aqueous phase respectively; concentrating the first ethyl ester phase to obtain syrup; dissolving the syrup in a methanol-water mixed solvent to obtain a sucralose-6-acetate methanol aqueous solution; the sucralose-6-acetate crude product water solution comprises sucralose-6-acetate, sucralose diester and tetrachlorosucrose-6-acetate;
(2) Mixing the sucralose-6-acetate methanol aqueous solution with an alkali metal hydroxide for alkaline hydrolysis reaction, and adjusting the pH value of the obtained reaction solution to be neutral to obtain a sucralose alkaline hydrolysis solution; concentrating the sucralose alkaline hydrolysis solution to obtain a sucralose crude product concentrate, mixing and dissolving the sucralose crude product concentrate and ethyl acetate, and performing solid-liquid separation to obtain a second ethyl ester phase;
(3) Washing the second ethyl ester phase with water to respectively obtain a second aqueous phase and a third ethyl ester phase; performing ethyl acetate extraction on the second aqueous phase to respectively obtain a third aqueous phase and a fourth ethyl ester phase; the fourth ethyl ester phase is recycled to step (2) for dissolving the crude sucralose concentrate;
(4) Mixing the third ethyl ester phase with water, and concentrating to obtain a third ethyl ester phase concentrate; dissolving the third ester phase concentrate in ethyl acetate to obtain a crude sucralose ethyl acetate solution; crystallizing the ethyl acetate solution of the sucralose crude product to respectively obtain a sucralose crude product and a fifth ethyl ester phase;
(5) Washing the fifth ethyl ester phase with water to obtain a fourth aqueous phase and a sixth ethyl ester phase respectively; the fourth aqueous phase is recycled to be mixed with the third ethyl ester in step (4); concentrating the sixth ethyl ester phase to respectively obtain recovered ethyl acetate and sugar residues.

In the present invention, unless otherwise specified, all the raw material components are commercially available products well known to those skilled in the art.

In the present invention, the sucralose-6-acetate crude product water solution is extracted by using ethyl acetate to obtain a first ethyl ester phase and a first aqueous phase respectively; the first ethyl ester phase is concentrated to obtain syrup; the syrup is dissolved in a methanol-water mixed solvent to obtain a sucralose-6-acetate methanol aqueous solution; the crude sucralose-6-acetate aqueous solution comprises sucralose-6-acetate, sucralose diester and tetrachlorosucrose-6-acetate.

In the present invention, the content of the sucralose-6-acetate in the raw material liquid is preferably 50-80 g/L, and more preferably 50-70 g/L; the mass ratio of the sucralose-6-acetate, the sucralose diester, and the tetrachlorosucrose-6-acetate in the raw material liquid is preferably 1: 0.06-0.15: 0.05-0.15, more preferably 1: 0.08-0.1: 0.06-0.1; the raw material liquid preferably also comprises NH₄Cl and organic impurities, and the concentration of NH₄Cl is preferably 80-150 g/L, and more preferably 85-120 g/L; the concentration of the organic impurities is preferably 30-80 g/L, and more preferably 30-50 g/L.

The preparation method of the crude sucralose-6-acetate aqueous solution is not particularly limited, and the crude sucralose-6-acetate aqueous solution with the components can be obtained by a preparation preparation method of the crude sucralose-6-acetate from sucrose, which is well known to those skilled in the art, and the preparation method comprises the following steps: preparing a solution containing sucrose-6-acetate by taking sucrose as a raw material, N, N-Dimethylformamide (DMF) as a solvent, organotin as a catalyst and acetic anhydride as an acylating agent; and then sequentially carrying out chlorination (thionyl chloride) and ammonia neutralization on the obtained solution containing the sucrose-6-acetate, carrying out vacuum concentration until the solution is dry, and dissolving by adding water to obtain a crude sucralose-6-acetate aqueous solution.

In the present invention, the temperature of ethyl acetate extraction is preferably 40-60 °C, more preferably 45-55 °C, and further preferably 50 °C; the number of times of ethyl acetate extraction is not particularly limited, as long as the sucralose-6-acetate content in an aqueous phase (namely the first aqueous phase) obtained by ethyl acetate extraction is < 0.1g/L, specifically 4-8 times; the time for single ethyl acetate extraction is preferably 10-30 min, and more preferably 15-25 min; the volume ratio of the crude sucralose-6-acetate aqueous solution to ethyl acetate for single ethyl acetate extraction is preferably 1: 0.2-0.6, more preferably 1: 0.4-0.5. In the present invention, the ethyl ester phases obtained by ethyl acetate extraction are combined into a first ethyl ester phase, and the raffinate phase obtained by the last ethyl acetate extraction is a first aqueous phase, wherein the first aqueous phase is preferably subjected to high-salt wastewater treatment.

The concentration method is not particularly limited in the invention, and the concentration method known by a person skilled in the art can be adopted, specifically, the concentration is carried out in vacuum, the concentration temperature is preferably 60-80 °C, the vacuum degree is preferably -0.1 to -0.08 MPa (gauge pressure), the concentration time is not particularly limited in the invention, and the concentration is carried out until the residual quantity of the ethyl acetate in the syrup is < 0.5 g/L. According to the method, the residual quantity of the ethyl acetate in the syrup is controlled, so that byproducts such as acetic acid and ethanol generated by the ethyl acetate in the subsequent alkaline hydrolysis step can be avoided, and the purity and the yield of the sucralose are further improved.

In the present invention, the concentration of methanol in the methanol-water mixed solvent is preferably 10-60 wt%, and more preferably 20-50 wt%. In the present invention, the concentration of the sucralose-6-acetate in the sucralose-6-acetate methanol aqueous solution is preferably 50-100 g/L, and more preferably 50-80 g/L.

After the sucralose-6-acetate methanol aqueous solution is obtained, mixing the sucralose-6-acetate methanol aqueous solution with an alkali metal hydroxide for an alkaline hydrolysis reaction, and adjusting the pH value of the obtained reaction solution to be neutral to obtain a sucralose alkaline hydrolysis solution; and concentrating the sucralose alkaline hydrolysis liquid to obtain a sucralose crude product concentrate, mixing and dissolving the sucralose crude product concentrate and ethyl acetate, and performing solid-liquid separation to obtain a second ethyl ester phase.

In the present invention, the alkali metal hydroxide preferably includes sodium hydroxide and/or potassium hydroxide; the alkali metal hydroxide is preferably used in the form of an aqueous alkali metal hydroxide solution, and the concentration of the aqueous alkali metal hydroxide solution is preferably 10-40 wt%, and more preferably 20-35 wt%; There is no special limitation on the dosage of the alkali metal hydroxide in the present invention, as long as it can ensure that the pH value during the alkaline hydrolysis reaction process is within the range of 12-12.5, more preferably 12.1-12.4, and further preferably 12.2-12.3; the temperature of the alkaline hydrolysis reaction is preferably 0-20 °C, more preferably 5-15 °C, and further preferably 5-10 °C; the time of the alkaline hydrolysis reaction is preferably 0.5-2 h, and more preferably 1-1.5 h. The invention carries out alkaline hydrolysis reaction under the above conditions, the sucralose diester can be hydrolyzed to generate sucralose, the tetrachlorosucrose-6-acetate can be dechlorinated and hydrolyzed to generate sucralose, and by-products generated by overhigh pH value of the alkaline hydrolysis reaction or overhigh temperature of the alkaline hydrolysis reaction can be avoided; the alkaline hydrolysis is carried out in a methanol-water system, so that the solubility of fat-soluble impurities in a water-containing system under a low-temperature condition can be increased, and the alkaline hydrolysis efficiency is improved. In addition, the invention takes alkali metal hydroxide as an alkaline hydrolysis reagent to carry out alkaline hydrolysis in a methanol-water system, and compared with the traditional sodium methoxide/methanol reaction system with higher hazard, the invention does not need to adopt high-purity alkali sucralose-6-acetate, omits the purification step of the sucralose-6-acetate, greatly shortens the flow and reduces the production cost.

In the present invention, the acid used to adjust the pH to neutrality preferably comprises hydrochloric acid; the concentration of the acid is preferably 15-35 wt%, more preferably 20-30 wt%. The using amount of the acid is not particularly limited, as long as the system can be neutralized to a pH value of 6.8-7.

In the present invention, the concentration method is not limited, and the concentration method known to those skilled in the art can be adopted, such as vacuum concentration; the concentration temperature is preferably 60-80 °C, the vacuum degree is preferably-0.1-0.08 MPa (gauge pressure), the concentration condition is not particularly limited, as long as the concentration is carried out until the water content of the sucralose crude product concentrate is < 0.1 wt%. According to the method, the residual quantity of the ethyl acetate in the sucralose-6-acetate aqueous solution is controlled, so that byproducts such as acetic acid and ethanol generated by the ethyl acetate in the subsequent alkaline hydrolysis step can be avoided, and the purity and the yield of the sucralose are further improved.

In the present invention, the volume ratio of the sucralose-6-acetate methanol aqueous solution to the ethyl acetate is preferably 1: 0.5-1, more preferably 1: 0.6-0.9.

In the present invention, the solid-liquid separation preferably includes filtration or suction filtration, and the purpose of the solid-liquid separation is to remove alkali metal salts and ammonium chloride; the alkali metal salt preferably comprises sodium chloride and/or potassium chloride. In the present invention, the content of inorganic salts in the second ethyl ester phase is preferably < 100 ppm.

After the second ethyl ester phase is obtained, the second ethyl ester phase is washed by water to respectively obtain a second aqueous phase and a third ethyl ester phase. In the present invention, the number of times of washing is preferably 5-7 times; the volume ratio of the second ethyl ester phase to the water-washed single-use water is preferably 0.1-0.3: 1, more preferably 0.15-0.25: 1, more preferably 0.2: 1; preferably, aqueous phases obtained by 1-2 times of washing are combined to be used as a second aqueous phase, the aqueous phase obtained by 2-7 times of washing is preferably used for washing the second ethyl ester phase in the next batch of sucralose crude product preparation process, specifically, the aqueous phase obtained by 3 times of washing is used for the first washing of the second ethyl ester phase in the next batch of sucralose crude product preparation process, the aqueous phase obtained by 4 times of washing is used for the second washing of the second ethyl ester phase in the next batch of sucralose crude product preparation process, the aqueous phase obtained by 5 times of washing is used for the third washing of the second ethyl ester phase in the next batch of sucralose crude product preparation process, the aqueous phase obtained by 6 times of washing is used for the fourth washing of the second ethyl ester phase in the next batch of sucralose crude product preparation process, and the aqueous phase obtained by 7 times of washing is used for the fifth washing of the next batch of second ethyl ester phase (namely, the aqueous phases obtained by 3-7 times of washing are sequentially used for the first to the fifth washing of the second ethyl ester phase in the next batch of sucralose crude product preparation process), and preferably, the 6 th to the 7 th washing of the second ethyl ester phase in the next batch of sucralose crude product preparation process are carried out by using pure water.

According to the invention, the second aqueous phase is preferably subjected to ethyl acetate extraction to obtain a third aqueous phase and a fourth ethyl ester phase respectively; the fourth ethyl ester phase is recycled to step (2) for dissolving the crude sucralose concentrate; the content of the sucralose in the third aqueous phase is preferably < 0.5 g/L; the third aqueous phase is preferably subjected to waste water treatment. In the present invention, the residual sucralose in the second aqueous phase enters the second ethyl ester phase and is recycled in step (2), so that the yield of the sucralose can be further improved.

After the third ethyl ester phase is obtained, mixing the third ethyl ester phase with water, and concentrating to obtain a third ethyl ester phase concentrate; dissolving the third ester phase concentrate in ethyl acetate to obtain a crude sucralose ethyl acetate solution; crystallizing the ethyl acetate solution of the sucralose crude product to respectively obtain a sucralose crude product and a fifth ethyl ester phase.

In the present invention, the volume ratio of the third ethyl ester phase to water is preferably 1: 0.1-0.3, more preferably 1: 0.2.

The concentration method is not particularly limited, and the concentration method known to a person skilled in the art can be adopted, specifically, vacuum concentration is adopted, the concentration temperature is preferably 60-80 °C, the vacuum degree is preferably -0.1 to -0.08 MPa (gauge pressure), the concentration time is not particularly limited, and the concentration is carried out until the water content of the triethyl ester phase concentrate is < 0.5 wt%.

The amount of the ethyl acetate is not particularly limited, and the sugar degree (Bx) of the ethyl acetate liquid of the sucralose crude product is 40-60%, more preferably 45-55%, and even more preferably 50%.

In the present invention, the crystallization temperature is preferably 40-60 °C, more preferably 45-55 °C, and further preferably 50 °C; the crystallization time is preferably 4-12 h, more preferably 5-10 h, and further preferably 6-8 h.

After the crystallization is completed, the method preferably further comprises solid-liquid separation to obtain a sucralose crude product and a fifth ethyl ester phase respectively. In the present invention, the solid-liquid separation preferably includes filtration or suction filtration.

After obtaining the fifth ethyl ester phase, the invention preferably further comprises; washing the fifth ethyl ester phase with water to obtain a fourth aqueous phase and a sixth ethyl ester phase respectively; said fourth aqueous phase is preferably recycled to step (4) for mixing with the third ester phase; the sixth ethyl ester phase is preferably concentrated to obtain recovered ethyl acetate and sugar residue, respectively. The water washing is not particularly limited, and the sucralose content in the sixth ethyl ester phase is < 0.1 g/L. In the present invention, the sugar residues are preferably subjected to solid waste treatment.

The water-soluble impurities and the fat-soluble impurities are generated in a series of reaction processes of the sucrose, so that they have a similar main body structure with sucralose, and the water-soluble impurities, the fat-soluble impurities and the sucralose have a certain mutual solubility. The invention uses the relationship between the three, and selects water and ethyl acetate as the solvent for removing water-soluble impurities and fat-soluble impurities, thereby using two solvents to dissolve and carry sucralose, realizing the exchange of sucralose in the two solvents, enriching sucralose in ethyl acetate, and crystallizing to obtain a sucralose crude product.

The technical solution of the present invention will be clearly and completely described below with reference to the embodiments of the present invention. It should be apparent that the described embodiments are only some embodiments of the present invention, and not all embodiments. All other embodiments, which can be obtained by a person skilled in the art without making any creative effort based on the embodiments in the present invention, belong to the protection scope of the present invention.

The contents of each substance in the following examples were measured by High Performance Liquid Chromatography (HPLC) using an external standard method under the following conditions: Japanese Shimadzu high performance liquid chromatograph, with RID-10A refractive index detection unit, LC-10ADVP high-pressure pump, CTO-10ASVP constant temperature box; chromatographic column: AgilentXDB C18 column (250 mm×4.6 mm, 5 µm); mobile phase: methanol-0.125 wt% aqueous dipotassium hydrogen phosphate (4:6, v/v); column temperature: 40 °C; mobile phase flow rate: 1.0 mL/min; wherein, methanol (chromatogram purity), dipotassium hydrogen phosphate (analytical purity), water is ultrapure water.

The aqueous solution of crude sucralose-6-acetate (designated as the first aqueous solution, the composition of which is shown in table 1) used in the following examples was prepared as follows: using sucrose as raw material, DMF as solvent, organotin as catalyst, acetic anhydride as acylating agent to prepare a solution containing sucrose-6-acetate; chlorinating the sucrose-6-acetate solution, neutralizing with ammonia water, concentrating in vacuum to dryness, and dissolving by adding water to obtain sucralose-6-acetate crude product aqueous solution.

**Table 1. Composition of the first aqueous solution**

| Components | Content |
|---|---|
| Sucralose-6-acetate | 58.7 g/L |
| Sucralose diester | 5.6 g/L |
| Tetrachlorosucrose-6-acetate | 4.3 g/L |
| NH₄Cl | 86.6 g/L |
| Other organic impurities | 30.1 g/L |
| Water | 82.4 wt% |

### Example 1

The process flow chart shown in FIG. 1 is adopted to prepare sucralose crude product, and the specific steps are as follows:
(1) Heating 1000 mL of the first aqueous solution to 50 °C, repeatedly extracting with ethyl acetate (the single dosage is 500 mL) for 4 times, and after the extraction is finished, combining ester phases to be used as a first ethyl ester phase and combining aqueous phases to be used as a first aqueous phase; the sucralose-6-acetate content in the first aqueous phase is 0.08 g/L, and the first aqueous phase is subjected to high-salt wastewater treatment; concentrating the first ethyl ester phase in vacuum to be dry (concentrate to dryness in short), and adding 50 wt% of methanol-water mixed solvent into the obtained syrup (the content of ethyl acetate is < 0.1 g/L) for dissolving to obtain 1000 mL of sucralose-6-acetate methanol aqueous solution.
(2) Cooling the aqueous solution of the sucralose-6-acetate methanol to 5 °C, then dropwise adding a 32% sodium hydroxide aqueous solution, carrying out alkaline hydrolysis reaction for 1 h at 5 °C and pH value of 12.3, wherein the content of the sucralose-6-ethyl ester is 0.4 g/L when the reaction is finished; then, adding hydrochloric acid with the concentration of 30 wt% dropwise to neutralize until the pH value is 7, concentrating the obtained sucralose alkaline hydrolysis solution to be dry, adding ethyl acetate into the obtained crude sucralose concentrate (the water content is 0.08 wt%) to dissolve(the volume ratio of the sucralose-6-acetic ester methanol aqueous solution to the ethyl acetate is 1: 0.6), and filtering to obtain a second ethyl ester phase, wherein the residual amount of sodium chloride in the second ethyl ester phase is 97 ppm.
(3) Washing the second ethyl ester phase with water for 5 times (the volume ratio of the second ethyl ester phase to the single-time water used for washing is 1:0.15), wherein the aqueous phases obtained by washing for 1-2 times are combined into a second aqueous phase, and all ester phases are combined into a third ethyl ester phase; and sequentially using the aqueous phase obtained by the third to fifth water washing for the first to third water washing of the second ethyl ester phase in the next batch of sucralose crude product preparation process. Adding ethyl acetate with the volume 2 times that of the second aqueous phase to extract, and layering to respectively obtain a third aqueous phase and a fourth ethyl ester phase; the sucralose content in the third aqueous phase is 0.3 g/L, and the third aqueous phase is subjected to wastewater treatment; the fourth ethyl ester phase is recycled to step (2) for dissolving the crude sucralose concentrate;
(4) Adding water into the third ethyl ester phase (the volume ratio of the third ethyl ester phase to water is 1: 0.2), concentrating to remove the water and the ethyl acetate, adding the ethyl acetate into the obtained third ethyl ester phase concentrate (the water content is 0.4 wt%), adjusting the sugar degree Bx to 55%, crystallizing at 45 °C for 10 h, and performing suction filtration to obtain a sucralose crude product and a fifth ethyl ester phase respectively;
(5) Adding pure water into the fifth ethyl ester phase to clean residual sugar until the content of sucralose is 0.07 g/L to obtain a fourth aqueous phase and a sixth ethyl ester phase respectively. The fourth aqueous phase is recycled to step (4) and is concentrated and dried together with the third ethyl ester phase; concentrating the sixth ethyl ester phase in vacuum to dryness to obtain recovered ethyl acetate and sugar residues respectively, and performing solid waste treatment on the sugar residues;
(6) The fourth ethyl ester phase, the fourth aqueous phase and the fifth aqueous phase (used for the water washing of the second ethyl ester phase) were recycled 17 times according to the operations of steps (1) to (5) (described as recycling 0 times), wherein "dissolving by adding ethyl acetate (volume ratio of sucralose-6-acetate methanol aqueous solution to ethyl acetate: 1: 0.6)" in step (2) was replaced by "dissolving by adding the second ethyl ester phase obtained in step (4) of example 1"; in step (3), the first to third water washing of the second ethyl ester phase sequentially utilizes the aqueous phase obtained by the third to fifth water washing in step (3) in the previous sucralose crude product preparation process, and the fourth to fifth water washing of the second ethyl ester phase is carried out by utilizing pure water; replacing "adding water to the third ethyl ester phase (volume ratio of the third ethyl ester phase to water = 1: 0.2)" in step (4) with "adding the fourth aqueous phase obtained in step (5) of example 1 to the third ethyl ester phase";

The purity and yield data of the crude sucralose product are shown in table 2.

**Table 2. Purity and yield of sucralose crude product**

| Recycling times | Mass of sucralose crude product/g | Purity/% | Mass of purified sucralose/g | Yield/% | Average/% |
|---|---|---|---|---|---|
| 0 | 27.81 | 90.12 | 25.06 | 47.21 | 80.89 |
| 1 | 45.57 | 91.33 | 41.62 | 78.39 | 110.12 |
| 2 | 56.75 | 89.27 | 50.66 | 95.43 | |
| 3 | 61.83 | 88.34 | 54.62 | 102.89 | |
| 4 | 64.06 | 90.15 | 57.75 | 108.77 | |
| 5 | 67.69 | 86.45 | 58.52 | 110.22 | |
| 6 | 64.06 | 91.34 | 58.51 | 110.21 | |
| 7 | 65.63 | 88.89 | 58.34 | 109.88 | |
| 8 | 66.22 | 87.66 | 58.05 | 109.34 | |
| 9 | 64.72 | 90.54 | 58.60 | 110.37 | |
| 10 | 64.76 | 91.00 | 58.93 | 111.00 | |
| 11 | 66.20 | 88.65 | 58.69 | 110.54 | |
| 12 | 62.89 | 92.01 | 57.87 | 109.00 | |
| 13 | 66.00 | 89.38 | 58.99 | 111.11 | |
| 14 | 65.14 | 89.43 | 58.26 | 109.73 | |
| 15 | 65.16 | 90.33 | 58.86 | 110.87 | |
| 16 | 65.52 | 89.34 | 58.53 | 110.25 | |
| 17 | 65.79 | 89.07 | 58.60 | 110.37 | |

| | | | | | |
|---|---|---|---|---|---|
| Note: the yield is the mass of sucralose finally obtained divided by the mass of sucralose-6-acetate completely converted to sucralose. Both the sucralose diester and the tetrachlorosucrose-6-acetate can be converted to sucralose after alkaline hydrolysis, with a theoretical maximum yield of 116.42% in example 1. | | | | | |

In the recycling process, the sucralose and the impurities are in an equilibrium state in the ethyl ester phase, namely recycling until the yield of the sucralose is more than 105%, and the system adopted by the invention is characterized in that: owing to the conversion of sucralose diester and tetrachlorosucrose-6-acetate, sucralose can achieve a yield of more than 110% (based on the conversion of sucralose-6-acetate to sucralose); the mutual washing and usage of the ester phase and the aqueous phase can avoid the loss caused by the residue of sucralose in sugar residue and wastewater to the greatest extent.

From the above examples, it can be seen that in the first few operation procedures, such as the recycling of first to fourth cycle, the yield is low because the recycled ethyl acetate and water in the system contain low components which can be converted into sucralose. After the sucralose is recycled used for 3 times, If the conversion of all sucralose-6-acetate into sucralose is used as the yield calculation method, the yield will exceed 100%, the reason is that other components (sucralose diester and tetrachlorosucrose-6-acetate) which can be converted into the sucralose are also included in the sucralose-6-acetate crude product aqueous solution, the method provided by the invention can convert the sucralose diester and tetrachlorosucrose-6-acetate into the sucralose, so that the yield of the sucralose is obviously improved, compared with a preparation method of the sucralose by using high-purity sucralose-6-acetate as a raw material through alkaline hydrolysis, the method provided by the invention has the advantage that the yield of the sucralose is obviously improved, and the sucralose-6-acetate crude product aqueous solution is used as the raw material, the method does not need to purify the product and has simpler process.

In addition, before ethyl acetate/water double-solvent extraction-back extraction, inorganic salt is separated from the system, so that the subsequent treatment difficulty of high-salinity wastewater is reduced. The solubility of the sucralose in the ethyl acetate is low, and in the process of recycling the fourth ester phase and the fourth aqueous phase, fat-soluble caramel impurities in the system are introduced into the ethyl acetate, so that the solubility of the sucralose in the ethyl acetate can be remarkably increased, and the impurities in the system are balanced in the methods of extraction, water washing and recycling, so that the sucralose can be enriched in the ethyl acetate and crystallized; by increasing the times of ethyl acetate/water double-solvent extraction-back extraction, the subsequent recovery purpose that partial mother liquor/water washing water containing the sucralose needs to return to the concentration step and be concentrated can be avoided. In addition, in the crystallization mother liquor (crude sucralose ethyl acetate liquor), since it is an ethyl acetate solvent system with more fat-soluble impurities and sucralose has a relatively high solubility in water, the uncrystallized sucralose can be recycled and reused by adopting a multi-time water washing method, and the fat-soluble impurities are removed. By implementing the method, the yield of the sucralose can be obviously improved. Therefore, compared with the preparation method of sucralose by using high-purity sucralose-6-acetate as a raw material through alkaline hydrolysis, the method provided by the invention has quite obvious advantages.

In conclusion, the method provided by the invention adopts alkali metal hydroxide to carry out alkaline hydrolysis, and the ethyl acetate/water double-system is used for extraction and impurity removal, so that the sucralose-6-acetate, the sucralose diester and the tetrachlorosucrose-6-acetate can be converted into the sucralose, and the sucralose is fully enriched and crystallized in the ethyl ester phase, thereby avoiding the loss caused by purification before the alkaline hydrolysis of the sucralose-6-acetate in the traditional process and the loss of some useful impurities, obviously improving the yield of the sucralose, creating greater value and having great industrial prospect.

The above description of the embodiments is only intended to facilitate the understanding of the method of the invention and its core idea. It should be noted that, for those skilled in the art, without departing from the principle of the present invention, it is possible to make various improvements and modifications to the present invention, and those improvements and modifications also fall within the scope of the claims of the present invention. Various modifications to these embodiments will be readily apparent to those skilled in the art, and the generic principles defined herein may be applied to other embodiments without departing from the spirit or scope of the invention. Thus, the present invention is not intended to be limited to the embodiments shown herein but is to be accorded the widest scope consistent with the principles and novel features disclosed herein.

## Claims

1. A preparation method of a sucralose crude product by using an alcohol-water alkaline hydrolysis system, **characterized by** comprising the following steps:
(1) Extracting crude sucralose-6-acetate aqueous solution with ethyl acetate to obtain a first ethyl ester phase and a first aqueous phase respectively; concentrating the first ethyl ester phase to obtain syrup; dissolving the syrup in a methanol-water mixed solvent to obtain a sucralose-6-acetate methanol aqueous solution; the sucralose-6-acetate crude product water solution comprises sucralose-6-acetate, sucralose diester and tetrachlorosucrose-6-acetate;
(2) Mixing the sucralose-6-acetate methanol aqueous solution with an alkali metal hydroxide for alkaline hydrolysis reaction, and adjusting the pH value of the obtained reaction solution to be neutral to obtain a sucralose alkaline hydrolysis solution; concentrating the sucralose alkaline hydrolysis solution to obtain a sucralose crude product concentrate, mixing and dissolving the sucralose crude product concentrate and ethyl acetate, and performing solid-liquid separation to obtain a second ethyl ester phase;
(3) Washing the second ethyl ester phase with water to obtain a second aqueous phase and a third ethyl ester phase respectively; performing ethyl acetate extraction on the second aqueous phase to obtain a third aqueous phase and a fourth ethyl ester phase respectively; the fourth ethyl ester phase is recycled to step (2) for dissolving the crude sucralose concentrate;
(4) Mixing the third ethyl ester phase with water, and concentrating to obtain a third ethyl ester phase concentrate; dissolving the third ester phase concentrate in ethyl acetate to obtain a crude sucralose ethyl acetate solution; crystallizing the ethyl acetate solution of the sucralose crude product to obtain a sucralose crude product and a fifth ethyl ester phase respectively;
(5) Washing the fifth ethyl ester phase with water to obtain a fourth aqueous phase and a sixth ethyl ester phase respectively; the fourth aqueous phase is recycled to be mixed with the third ethyl ester in step (4); concentrating the sixth ethyl ester phase to obtain recovered ethyl acetate and sugar residues respectively.

2. The preparation method according to claim 1, wherein in step (1), the residual amount of ethyl acetate in the syrup is < 0.5 g/L.

3. The preparation method according to claim 1, wherein in step (1), the sucralose-6-acetate content in first aqueous phase is < 0.1g/L.

4. The preparation method according to claim 1, wherein in step (1), the concentration of methanol in the methanol-water mixed solvent is 10-60 wt%.

5. The preparation method according to claim 1, wherein in step (2), the pH value of the alkaline hydrolysis reaction is 12.0-12.5, the temperature is 0-20 °C, and the time is 0.5-2 h.

6. The preparation method according to claim 1 or 5, wherein in step (2), the water content in the crude sucralose concentrate is < 0.1 wt%.

7. The preparation method according to claim 1 or 5, wherein in step (2), the content of inorganic salts in the second ethyl ester phase is < 100 ppm.

8. The preparation method according to claim 1, wherein in step (3), the number of times of water washing is 5-7; the volume ratio of the second ethyl ester phase to the water used for single-time water washing is 0.1-0.3: 1; combining the aqueous phases obtained by the first to the second water washing to be used as a second aqueous phase;
The aqueous phase obtained by the second-seventh washing is used for washing the second ethyl ester phase in the next batch of sucralose crude product preparation process.

9. The preparation method according to claim 1 or 8, wherein in step (3), the sucralose content in the third aqueous phase is < 0.5 g/L.

10. The preparation method according to claim 1, wherein in step (4), the water content in the third ethyl ester phase concentrate is < 0.5 wt%.

11. The preparation method according to claim 1 or 10, wherein in step (4), the sugar degree of the ethyl acetate solution of the sucralose crude product is 40-60%.

12. The preparation method according to claim 1, wherein in step (4), the crystallization temperature is 40-60 °C, and the time is 4-12 h.

13. The preparation method according to claim 1, wherein in step (5), the sucralose content in the sixth ethyl ester phase is < 0.1 g/L.
